# EUROPEAN PATENT APPLICATION

(11) **EP 2 657 232 A2**
(43) Date of publication of application: **30.10.2013**
(21) Application number: 13164103.7
(22) Date of filing: 17.04.2013
(51) Int. Cl.: C07D 403/04

(54) **Process for the purification of a benzenesulphonamide compound**

(30) Priority: 27.04.2012 IT MI20120701
(71) Applicant: Dipharma Francis S.r.l., 20021 Baranzate (MI) (IT)
(72) Inventor: Razzetti, Gabriele, 20021 BARANZATE (MI) (IT); Allegrini, Pietro, 20021 BARANZATE (MI) (IT); Rossi, Davide, 20021 BARANZATE (MI) (IT)
(74) Representative: Bertuccio, Silvia

(57) **Abstract**

Process for the purification of N-[6-(2-hydroxy-ethoxy)-5-(2-methoxy-phenoxy)-2-pyrimidin-2-yl-pyrimidin-4-yl]-4-tert-butyl-benzenesulphonamide (bosentan), intermediates useful in said process, and their preparation.

## Description

### FIELD OF INVENTION

The present invention relates to a process for the purification of *N*-[6-(2-hydroxy-ethoxy)-5-(2-methoxy-phenoxy)-2-pyrimidin-2-yl-pyrimidin-4-yl]-4-tert-butyl-benzenesulphonamide (bosentan), intermediates useful in said process, and their preparation.

### PRIOR ART

Bosentan or *N*-[6-(2-hydroxy-ethoxy)-5-(2-methoxy-phenoxy)-2-pyrimidin-2-yl-pyrimidin-4-yl]-4-tert-butyl-benzenesulphonamide of formula **(I)** 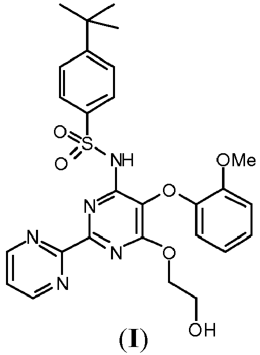 is an endothelin receptor antagonist known from US 5,292,740.

Bosentan is in the monohydrate crystalline form, as obtainable according to example 8 of US 6,136,971.

Methods for the preparation of bosentan of formula **(I)** were disclosed in US 5,292,740 and US 6,136,971.

The main drawback of the process disclosed in US 5,292,740 is the formation of the two known impurities described in Organic Process Research & Development, 2002, 6, 120-124, designated as **(13)** and **(12),** which have the following formulas

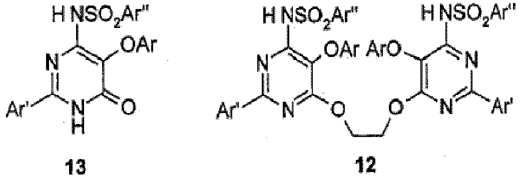

To eliminate said impurities, otherwise present in the API (Active Pharmaceutical Ingredient), laborious operations of purification of the final product are required, involving considerable practical and economic disadvantages.

The synthesis method disclosed in US 6,136,971 also entails the formation of impurities in the final product, in particular the compound of formula **(13)** reported above and impurity **(18)** reported below

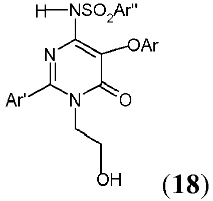

WO 2012/041764 attempts to solve the problem of eliminating the impurities of formula 13 and 12 by using a purification process which, though efficient, still does not drastically reduce the level of said impurities. Moreover, no mention is made of a process that would provide bosentan, in particular in the monohydrate form, with high purity, not containing other by-products.

In addition to the impurities reported above, numerous other more or less identified impurities exist.

A number of polymorphs of bosentan in unsalified form exist, such as the anhydrous or solvated crystalline forms known from WO 2008/135795 or WO 2009/083739. Bosentan in amorphous form is disclosed (*inter alia*) in WO 2008/135795. In addition to the problems involved in elimination of impurities, the low solubility of the various known crystalline forms of bosentan in the different solvents/solvent mixtures makes its purification by currently known processes complex and industrially impractical. For this reason, there is a need to provide a simple, efficient, industrially scalable process which produces bosentan, in particular in the monohydrate form, with the highest possible yield and as free of impurities as possible.

WO 2011/058524 discloses crystalline forms of bosentan sodium salt as such, which are not specifically suitable to obtain bosentan in the monohydrate crystalline form with both high yields and high purity.

### SUMMARY OF THE INVENTION

An advantageous process for the purification of bosentan has now been found which involves the use of specific anhydrous or solvated crystalline forms of bosentan sodium salt, defined herein as crystalline forms ω, ψ, χ, σ, ϕ and τ.

### BRIEF DESCRIPTION OF FIGURES AND ANALYSIS METHODS

The various crystalline modifications of bosentan sodium salt have been characterised by X-ray powder diffraction (XRPD) and differential scanning calorimetry (DSC). The water content of the compounds was determined by titration with the Karl Fischer technique. The X-ray diffraction spectra (XRPD) were collected with the Ital-Structures APD-2000 automatic powder and liquid diffractometer under the following operating conditions: CuKα radiation (λ = 1.5418 Å), scanning with a 2θ angle range of 3-40° with a step size of 0.03° for 1 sec. The DSC thermograms were acquired with a Mettler-Toledo DSC 822e differential scanning calorimeter, under the following operating conditions: open aluminium capsule, range 30-300°C at the rate of 10°C/min, with nitrogen as purge gas (80 ml/min).
Figure 1: XRPD spectrum of Bosentan Sodium Form ω
Figure 2: XRPD spectrum of Bosentan Sodium Form ψ
Figure 3: XRPD spectrum of Bosentan Sodium Form χ
Figure 4: XRPD spectrum of Bosentan Sodium Form σ
Figure 5: XRPD spectrum of Bosentan Sodium Form ϕ
Figure 6: XRPD spectrum of Bosentan Sodium Form τ
Figure 7: XRPD spectrum of Bosentan Sodium Form D described in WO 2011/058524.
Figure 8: DSC thermogram of Bosentan Sodium Form ω
Figure 9: DSC thermogram of Bosentan Sodium Form ψ
Figure 10: DSC thermogram of Bosentan Sodium Form χ
Figure 11: DSC thermogram of Bosentan Sodium Form σ
Figure 12: DSC thermogram of Bosentan Sodium Form ϕ
Figure 13: DSC thermogram of Bosentan Sodium Form τ

The size of the particles and, for example, the average size of the D₅₀ particles, are determined by the well-known laser light scattering technique, using a Malvern Mastersizer MS1 instrument under the following operating conditions:
- 300RF mm lens with 2.4 mm laser beam length;
- 500 mg sample dispersed in 10 ml of hexane (ACS reagent) with 1% of SPAN 85^{®}, without pre-sonication, and with a stirring rate of 2500 rpm.

### DETAILED DESCRIPTION OF THE INVENTION

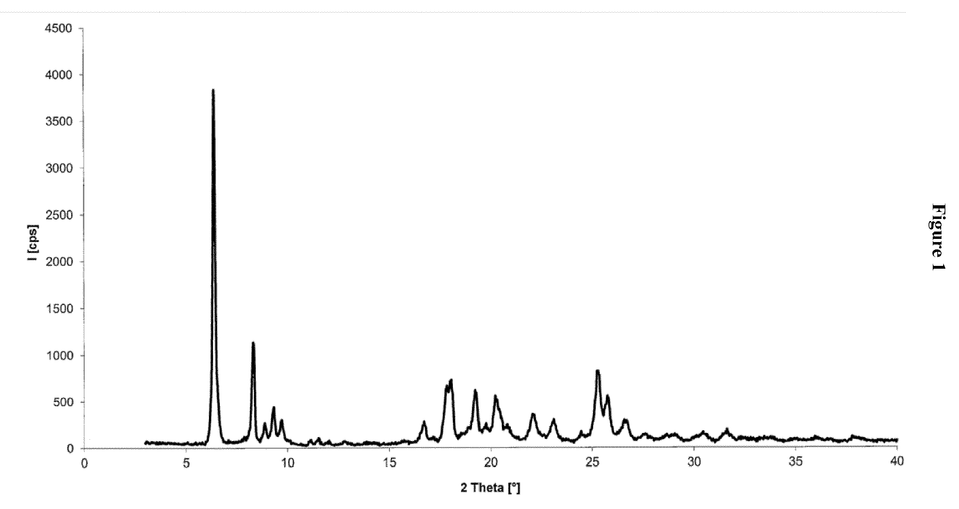

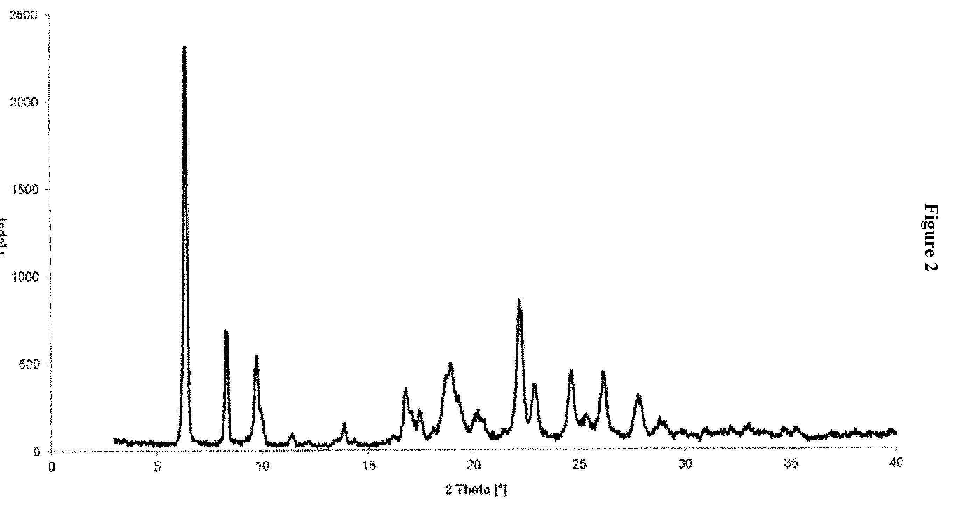

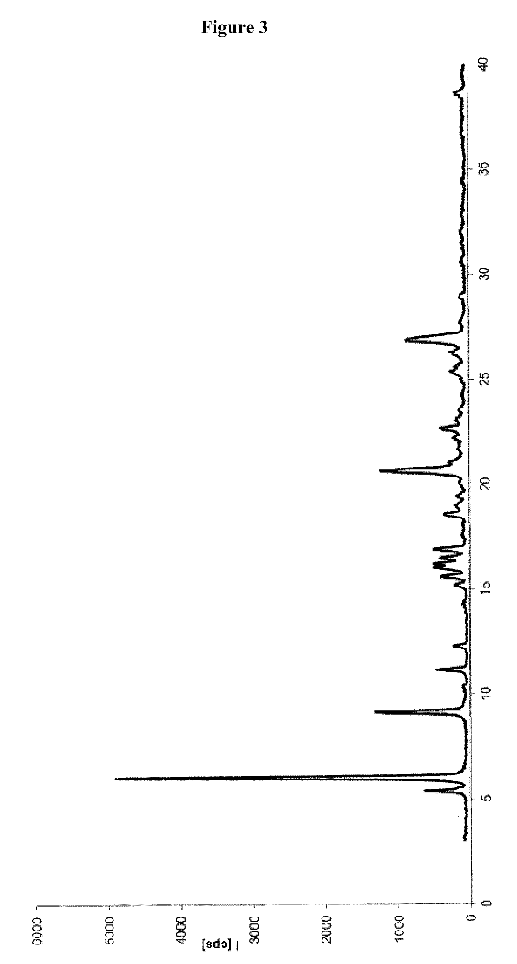

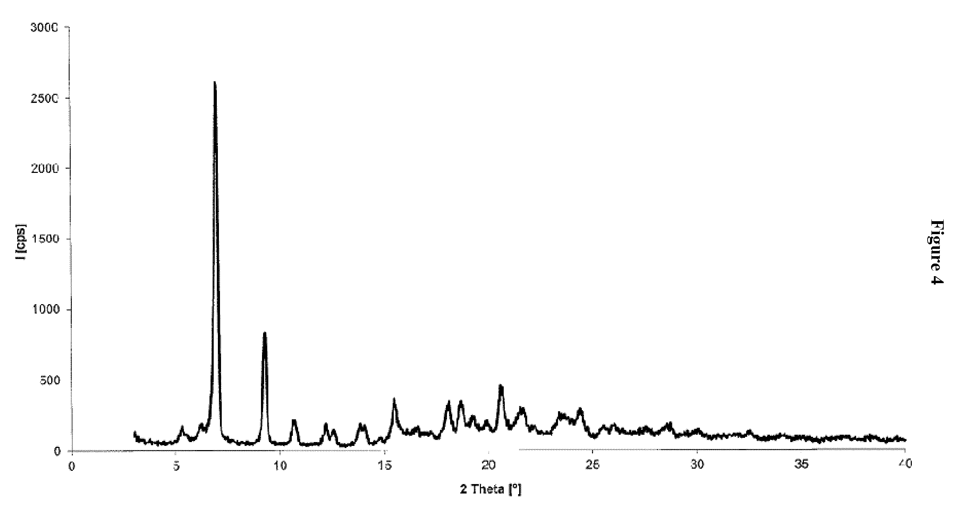

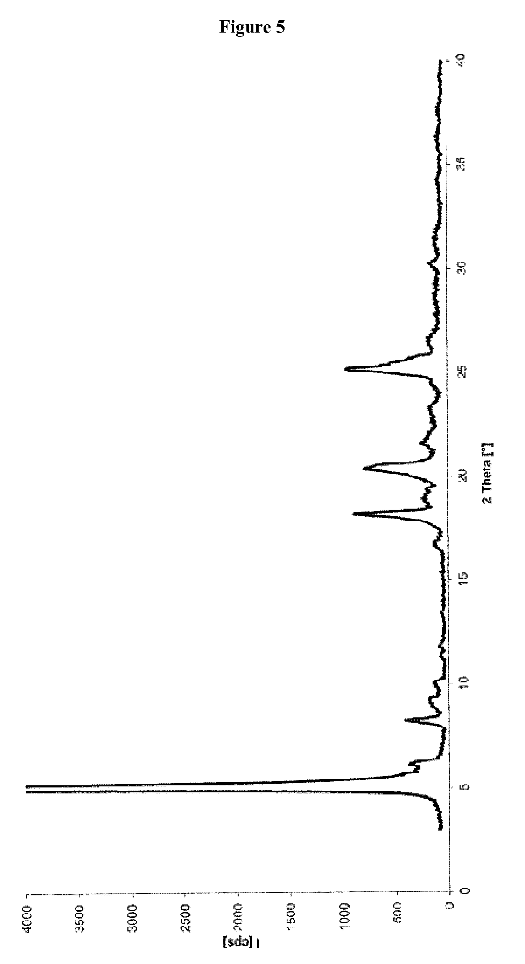

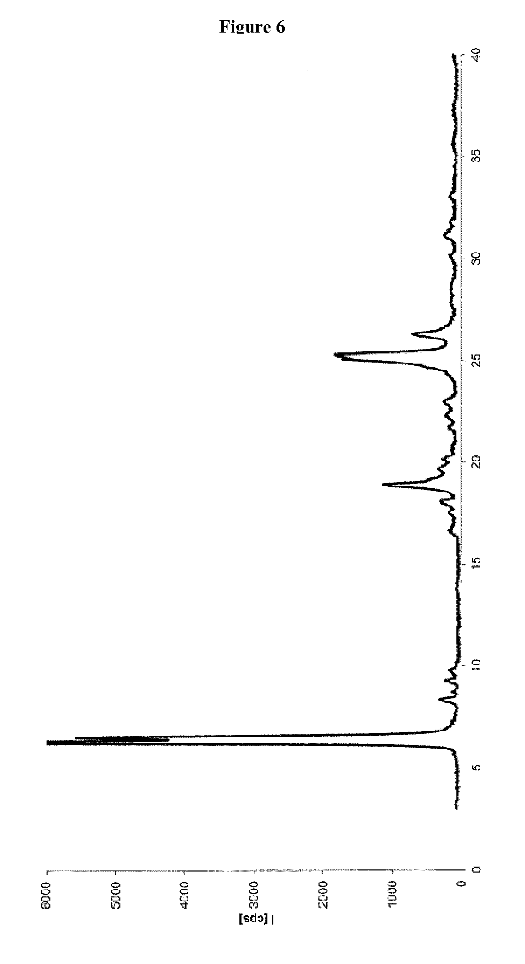

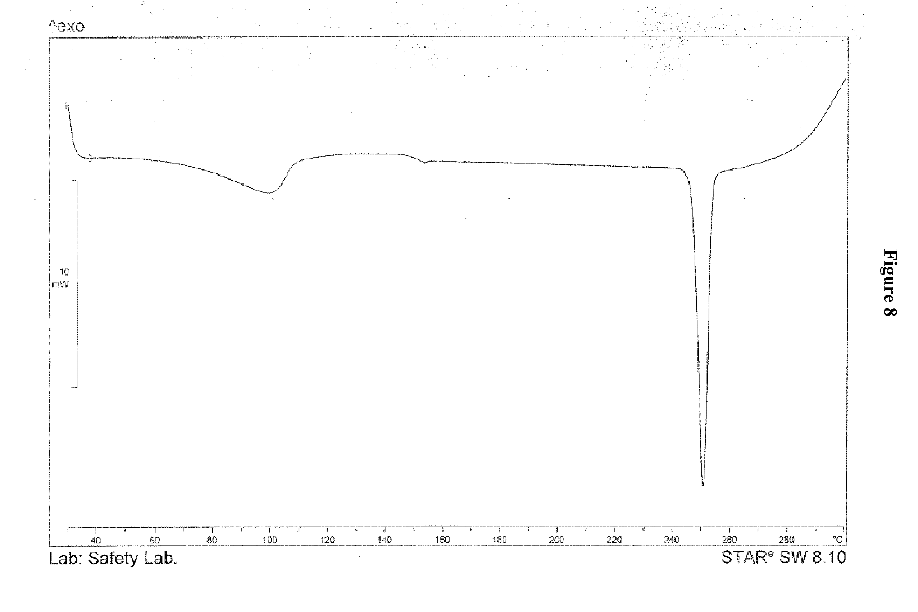

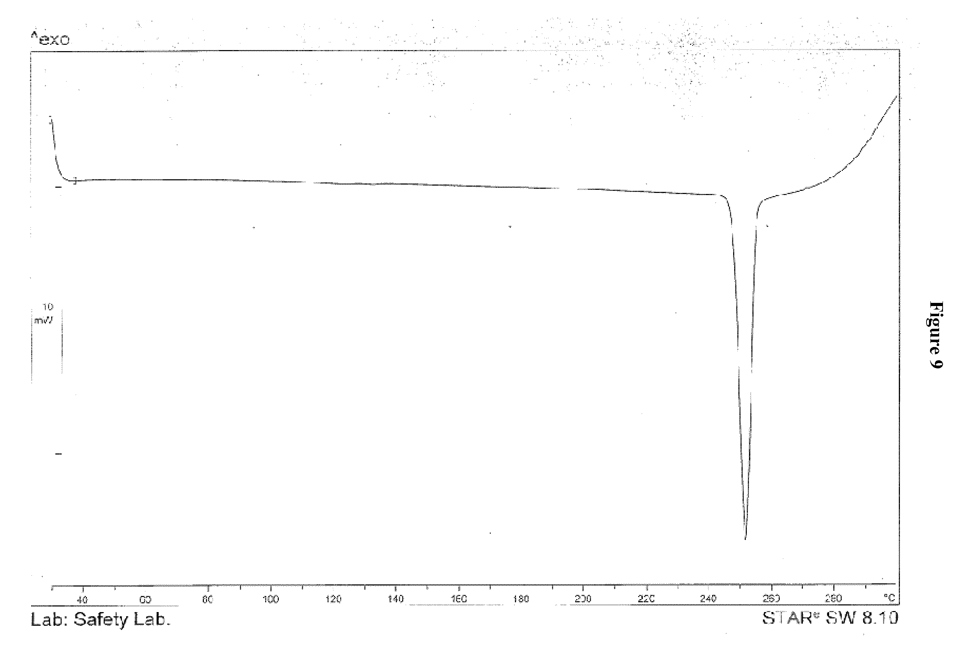

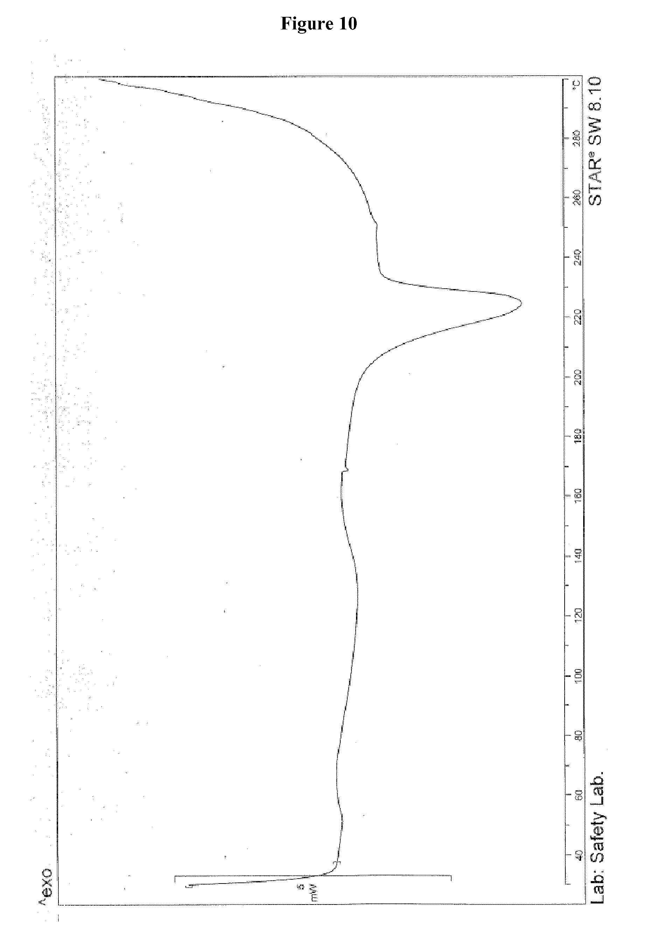

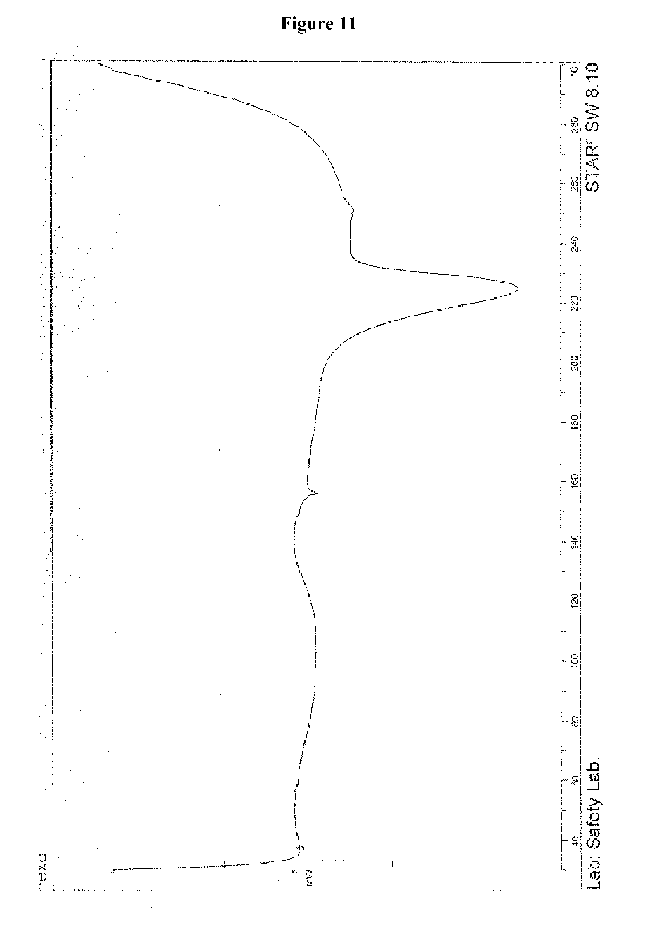

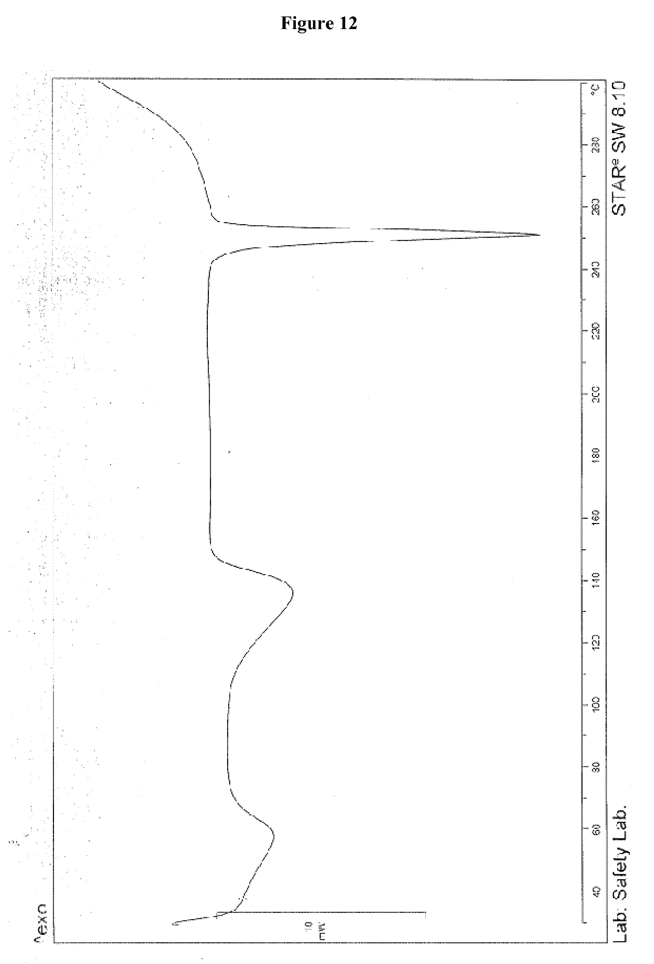

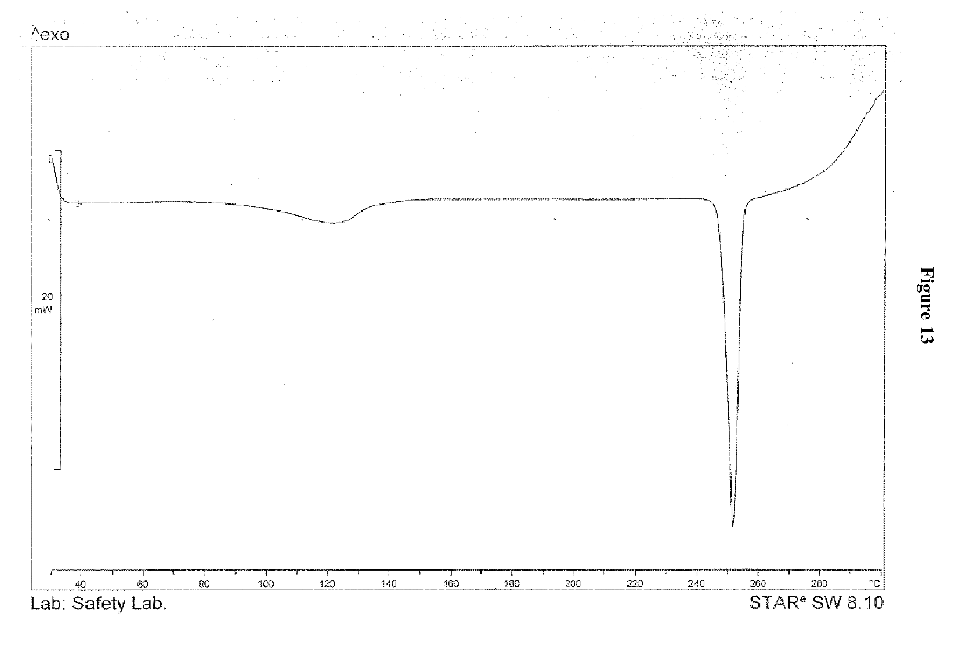

The object of the present invention is a process for the purification of bosentan of formula **(I)**, in particular in the monohydrate form, or a salt thereof, 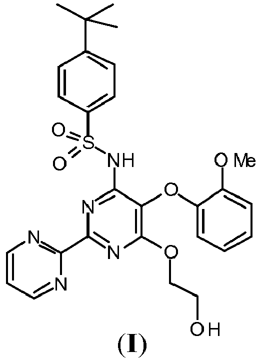 comprising:
a) preparing a crystalline form of bosentan sodium salt of formula **(I')** 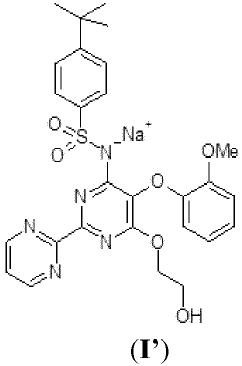 selected from the group comprising:
   i. a crystalline form, herein called Form ω, solvated with acetone and methanol, having a XRPD as substantially reported in Figure 1, wherein the most intense peaks fall at 6.39, 8.31; 8.88; 9.30; 9.69; 16.71; 17.82; 18.03; 19.23; 20.22; 22.05; 23.07; 25.26; 25.77; 26.58± 0.2° in 2θ and a DSC thermogram as substantially reported in Figure 8, wherein the main endothermic peaks fall at about 70°C-110°C and 246°C - 248°C;
   ii. a crystalline form, herein called Form ψ, having a XRPD as substantially reported in Figure 2, wherein the most intense peaks fall at 6.39; 8.28; 9.72; 11.4; 13.86; 16.77; 17.43; 18.12; 18.90; 10.22; 22.20; 22.86; 24.63; 26.16; 27.78± 0.2° in 2θ; and having a DSC thermogram as substantially reported in Figure 9, wherein the melting endotherm falls at about 246°C-248°C;
   iii. a crystalline form, herein called Form χ, having a XRPD as substantially reported in Figure 3, wherein the most intense peaks fall at 5.34; 6.03; 9.12; 11.16; 12.27; 15.18; 15.57; 16.02; 16.20; 16.47; 16.89; 18.54; 20.64; 22.65; 26.85 ± 0.2° in 2θ and having a DSC thermogram as substantially reported in Figure 10, wherein the melting endotherm falls at about 210°C-230°C;
   iv. a crystalline form, herein called Form σ, having a XRPD as substantially reported in Figure 4, wherein the most intense peaks fall at 5.31, 6.18; 6.96; 9.27; 10.68; 12.21; 12.57; 13.83; 15.48; 18.09; 18.69; 19.23; 20.61; 21.69; 24.39 ± 0.2° in 2θ and having a DSC thermogram as substantially reported in Figure 11, wherein the melting endotherm falls at about 210°C-230°C;
   v. a crystalline form, herein called Form ϕ, solvated with tetrahydrofuran, having a XRPD as substantially reported in Figure 5, wherein the most intense peaks fall at 4.95; 6.09; 8.16; 9.12; 9.99; 16.44; 17.94; 18.69; 20.28; 21.51; 22.83 and 24.93 ± 0.2° in 2θ; and a DSC tracing as substantially reported in Figure 12, wherein the main endothermic peaks fall at about 100°C-150°C and about 246°C - 248°C; and
   vi. a crystalline form, herein called Form τ, solvated with tetrahydrofuran, having a XRPD as substantially reported in Figure 6, wherein the most intense peaks fall at 6.27; 6.51; 8.34; 9.27; 9.78; 17.52; 18.09; 18.87; 19.68; 20.13; 21.69; 22.23; 22.98; 25.26; 26.28 ± 0.2° in 2θ; a DSC thermogram as substantially reported in Figure 13, wherein the main endothermic peaks fall at about 80°C-130°C and about 246°C - 248°C;
b) forming a dispersion of a crystalline form of bosentan sodium as defined above in ethanol, optionally mixed with water;
c) treating a dispersion thus obtained with an organic or inorganic acid, or a solution of said acid; and
d) recovering of bosentan of formula **(I),** in particular in the monohydrate form, and optionally, conversion to a salt thereof.

Bosentan of formula **(I),** in the monohydrate crystalline form, has the same chemico-physical characteristics as the product obtainable according to example 8 of US 6,136,971, and has a DSC and XRPD as reported in Figures 1 and 2 of EP 2 072 503.

A bosentan salt of formula **(I)** is preferably a pharmaceutically acceptable salt.

Bosentan sodium salt in crystalline form ϕ can be prepared by a process which comprises:
- forming a solution of bosentan sodium salt in tetrahydrofuran or a mixture thereof with at least one other solvent;
- cooling of the solution thus obtained, to obtain a precipitate;
- recovering the precipitate thus obtained.

The solution of bosentan sodium salt is preferably obtained by dissolving, for example, crude bosentan sodium salt in a solvent mixture comprising tetrahydrofuran and an additional solvent such as water.

The solvent mixture preferably comprises water and tetrahydrofuran.

The ratio in volume between tetrahydrofuran and water can typically be between 2:1 and 10:1, preferably between about 4:1 and about 8:1, and more preferably about 6:1.

The solution of bosentan sodium salt can be formed by heating in a temperature range between about 40°C and the reflux temperature of the solution.

To obtain a precipitate of bosentan sodium salt in crystalline form ϕ, the solution thus obtained can be cooled, typically in a temperature range between about 0°C and 50°C, more preferably between about 0 and 10°C, generally in a time of not less than 12 hours, and optionally with a cooling rate of between about 0.05 and 0.1°C/min; once the temperature has been reached, it is maintained for at least 4-6 hours. Precipitation can optionally be induced by seeding with a seed crystal of crystalline form ϕ previously obtained.

The crystalline product in Form ϕ can be recovered according to known techniques, such as filtration or centrifugation, preferably filtration.

Bosentan sodium salt crystalline form ϕ, thus obtained, has a degree of purity equal to or greater than 99%, in particular equal to or greater than 99.5%, and is characterised by an XRPD spectrum as defined above and substantially illustrated in Figure 5, and a DSC thermogram as reported in Figure 12.

Crude bosentan sodium salt can be obtained according to the process described in EP 2 072 503, example 7.

Bosentan sodium salt in crystalline form τ, as defined above, can be prepared by a process which comprises drying of bosentan sodium salt in crystalline form ϕ as defined above.

Bosentan sodium salt crystalline form ϕ can be dried at a temperature between about 30°C and about 70°C, preferably between about 45°C and about 50°C, typically at low pressure, in a interval of between about 5 and about 20 hours.

Bosentan sodium salt crystalline form τ, thus obtained, has a degree of purity equal to or greater than 99%, in particular equal to or greater than 99.5%, and is characterised by an XRPD spectrum as reported above and substantially illustrated in Figure 6, and a DSC thermogram as reported in Figure 13.

Bosentan sodium salt in crystalline form ω, as defined above, can be prepared by a process which comprises:
- dispersing bosentan sodium salt in crystalline form τ, as defined above, in a solvent mixture comprising at least acetone and methanol to obtain a dispersion of bosentan sodium salt;
- cooling of the dispersion;
- recovering the solid thus obtained.

The dispersion of bosentan sodium salt can be formed by heating the solvent mixture containing crystalline form τ at a temperature between about 30°C and the reflux temperature of the solvent mixture, preferably between about 40°C and the reflux temperature of the solvent mixture.

According to a preferred aspect, the solvent mixture only contains acetone and methanol.

The ratio in volume between acetone and methanol is typically between about 1:1 and about 8:1.

To obtain bosentan sodium salt in crystalline form ω, the solution thus obtained can be cooled, for example, in a temperature range between about 0°C and 50°C, more preferably between about 15 and 25°C, in a time generally not less than 2-4 hours, and optionally with a cooling rate of between about 0.1°C/min and 1°C/min; once the temperature has been reached, it is maintained for at least 12-14 hours.

The dispersion can then be further cooled to about 0°C in 1-2 hours, and maintained at said temperature for about 1-2 hours.

Alternatively, crystalline form ω can be prepared by seeding in a dispersion containing bosentan sodium salt, for example in crystalline form D, in a solvent as defined above, with a seed crystal of form ω as previously obtained, or seeding with a seed crystal of form ψ, as defined above, operating as reported above.

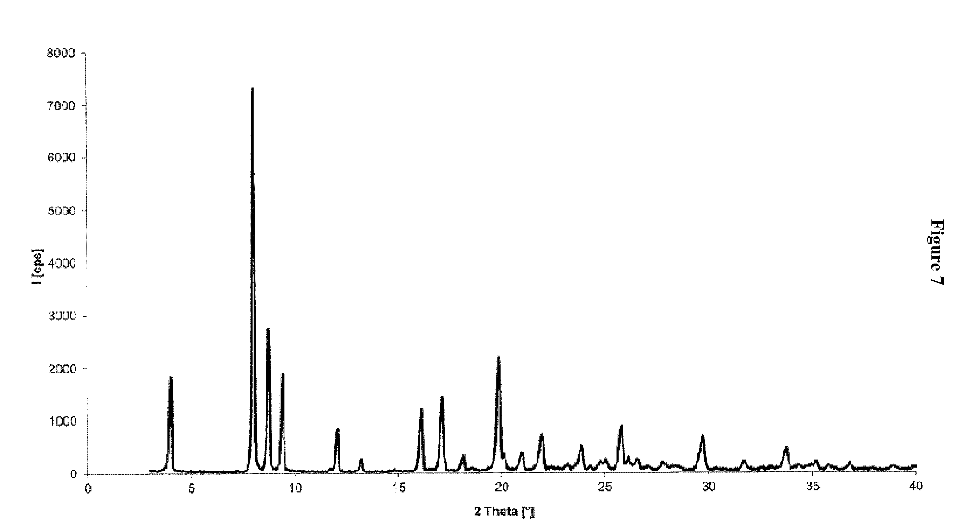

The XRPD and DSC of crystalline form D of bosentan sodium salt are reported in Figures 10 and 12 respectively of WO 2011/058524. The same XRPD data are reported here in Figure 7.

Said crystalline form D can also be obtained according to example 7 of EP 2 072 503.

The crystalline product in form ω can be recovered by known techniques such as filtration or centrifugation, preferably filtration.

Bosentan sodium salt crystalline form ω thus obtained has a degree of purity equal to or greater than 99%, in particular equal to or greater than 99.5%, and is characterised by an XRPD spectrum as defined above and as substantially illustrated in Figure 1, and a DSC thermogram as reported in Figure 8.

Bosentan sodium salt in crystalline form ψ can be prepared by a process which comprises drying bosentan sodium salt in crystalline form ω, as defined above.

Bosentan sodium salt crystalline form ω can be dried, for example, at a temperature between about 30°C and about 70°C, preferably between about 45°C and about 50°C, typically at low pressure, for example under vacuum, in a time ranging between about 5 and about 20 hours.

Bosentan sodium salt crystalline form ψ, thus obtained, has a degree of purity equal to or greater than 99%, in particular equal to or greater than 99.5%, and is characterised by an XRPD spectrum as defined above and as substantially illustrated in Figure 2, and a DSC thermogram as reported in Figure 9. Said form presents a water content of between about 0.1 and 1.0%, so that it can be described as substantially anhydrous;

Bosentan sodium salt crystalline form χ can be prepared by a process comprising:
- dispersing bosentan sodium salt in crystalline form D in a solvent mixture comprising a C₃-C₇ ketone and a C₁-C₇ alkanol;
- cooling of the dispersion;
- recovering the solid thus obtained.

A straight or branched C₃-C₇ ketone and a straight or branched C₁-C₇ alkanol are preferably acetone and methanol respectively.

The dispersion of bosentan sodium salt in crystalline form D can be effected by heating the solvent mixture at a temperature between about 30°C and the reflux temperature of the solvent mixture, preferably between about 40°C and the reflux temperature of the solvent mixture.

According to a preferred aspect of the invention, the solvent mixture as defined above is formed by acetone and methanol.

The ratio in volume between a straight or branched C₃-C₇ ketone and a straight or branched C₁-C₇ alkanol is typically between about 1:1 and about 8:1.

The dispersion can typically be cooled in a temperature interval between about 0°C and 50°C, more preferably between about 15 and 25°C, in a time generally not less than 2-3 hours, at a cooling rate typically between about 0.1 °C/min and 1.0°C/min; once the temperature has been reached, it is maintained for at least 12-14 hours.

The dispersion can then be further cooled to about 0°C in 1-2 hours, and maintained at said temperature for about 1-2 hours.

Alternatively, crystalline form χ can be prepared by seeding it with a seed crystal of crystalline form χ, previously obtained.

The crystalline product in form χ can be recovered by known techniques such as filtration or centrifugation, preferably filtration.

Bosentan sodium salt crystalline form χ thus obtained has a degree of purity equal to or greater than 99%, in particular equal to or greater than 99.5%, and is characterised by an XRPD spectrum as defined above and as substantially illustrated in Figure 3, and a DSC thermogram as reported in Figure 10.

Bosentan sodium salt crystalline form σ can be prepared by a process which comprises drying bosentan sodium salt crystalline form χ, as defined above.

Bosentan sodium salt crystalline form χ can be dried at a temperature between about 30°C and about 70°C, preferably between about 45°C and about 50°C, typically at low pressure, preferably under vacuum, in a time ranging between 5 and 20 hours.

Bosentan sodium salt crystalline form σ, thus obtained, has a degree of purity equal to or greater than 99%, in particular equal to or greater than 99.5%, and is characterised by an XRPD spectrum as defined above and as substantially illustrated in Figure 4, and a DSC thermogram as reported in Figure 11; said form presents a water content of between about 0.1 and 1.0%, so that it can be described as substantially anhydrous.

According to step b) of the process of the present invention, a dispersion of a crystalline form of bosentan sodium salt in ethanol, optionally mixed with water, can be formed at a temperature of between about 0°C and the reflux temperature of the solvent.

According to step c) of the process of the present invention an organic acid is, for example, a C₁-C₈ carboxylic acid, typically formic, acetic, propionic, succinic, fumaric or tartaric acid, or a sulphonic acid, such as methanesulphonic or ethanesulphonic acid.

An inorganic acid is, for example, sulphuric acid or a hydrohalic acid, in particular hydrochloric or hydrobromic acid.

Said acid is preferably an inorganic acid, more preferably 37% hydrochloric acid.

According to step d) of the process of the invention, bosentan of formula **(I)** can be recovered, for example, by filtering the solid through a Büchner filter or by other known methods.

Bosentan of formula **(I),** obtainable according to the purification process of the present invention, is bosentan in free, unsalified form, preferably in the monohydrate crystalline form, as obtainable according to example 8 of US 6,136,971, and has a DSC and XRPD as reported in Figures 1 and 2 of EP 2 072 503.

Bosentan of formula **(I),** thus obtained, can be converted to a salt thereof, typically a pharmaceutically acceptable salt, by known methods, such as by treatment with an organic or inorganic acid, or a solution thereof in a suitable solvent, such as one of those specified above.

According to a preferred aspect of the invention, the process for purification of bosentan of formula **(I),** in particular in the monohydrate crystalline form, described above, comprises the use as starting compound of a crystalline form of bosentan sodium salt selected from the group comprising the novel crystalline forms ω, ψ, ϕ and τ; more preferably form ω or ϕ.

It has been found that the purification process according to the invention allows bosentan of formula **(I)** to be obtained, in particular in the monohydrate crystalline form, with both high yields and high purity.

This unexpected result was achieved in particular by using one of the novel crystalline forms of bosentan sodium salt reported above, in particular the novel crystalline forms ω, ψ, ϕ and τ, more specifically the novel form ω or ϕ.

Bosentan of formula **(I)** or a salt thereof, in particular bosentan in the known monohydrate crystalline form, as obtainable according to the purification process of the invention, has a purity equal to or greater than 99.8%, in particular equal to or greater than 99.9%, and presents a content of impurity (12) lower than 0.05% and content of impurity (13) lower than the instrument detection limit.

It also has a mean particle diameter D₅₀ of between about 10 and 250 microns, preferably about 15-50 microns. If desired, said value can be reduced by micronisation or fine grinding.

Said high-purity result, which was entirely unexpected, relating to bosentan of formula **(I)** in the monohydrate form obtained by the process according to the present invention, is directly related with the high purity of the crystalline forms according to the invention.

Conversely, crystalline form D of bosentan sodium salt as obtainable according to examples 11 and 12 of WO 2011/058524 has a purity not exceeding 98.65%. Hence bosentan form **(I)** in the monohydrate form, if obtained by cleaving the sodium salt in an acid environment from said crystalline form D having a purity not exceeding 98.65%, cannot present a purity exceeding 99% because the salt cleavage procedure has no effect on the purification of the final product.

According to a further aspect, the invention therefore provides a crystalline form of bosentan sodium salt selected from the group comprising ω, ψ, χ, σ, ϕ and τ, as defined above. Said crystalline forms are novel, and are a further object of the invention.

The size of the crystals of bosentan sodium salt crystalline forms ω, ψ, χ, σ, ϕ and τ, as obtained, has a D₅₀ value of between about 10 and 250 µm. If desired, said value can be reduced by micronisation or fine grinding.

Another object of the present invention is a pharmaceutical composition containing at least one of the crystalline forms defined here as Form ω, ψ, χ, σ, ϕ and τ, and a pharmaceutically acceptable excipient and/or carrier. Said pharmaceutical composition can be prepared in a pharmaceutical form according to known methods. The dose of active ingredient present in said composition can be the same as commonly used in clinical practice for bosentan.

The examples below illustrate the invention.

### Example 1: Preparation of bosentan sodium salt crystalline form ϕ

30 g of bosentan sodium salt, 72 ml of tetrahydrofuran and 18 ml of deionised water are loaded into a 250 ml jacketed reactor. The mixture is heated at reflux temperature until complete dissolution; the mixture is then filtered hot through a Büchner funnel, and the filter is washed with 5 ml of tetrahydrofuran. The filtered solution is returned to the reactor and heated to 40°C, and crystallisation of the product is awaited; the mixture is then heated to 50°C and maintained under stirring at that temperature for 3 hours. The mixture is cooled to 0°C in 12 hours, and maintained at that temperature for 4 hours. The product is filtered and washed twice with 10 ml of tetrahydrofuran cooled to 0°C. Yield: 95%. The crystalline solid presents a XRPD as substantially reported in Figure 5, wherein the most intense peaks are observed at 4.95; 6.09; 8.16; 9.12; 9.99; 16.44; 17.94; 18.69; 20.28; 21.51; 22.83 and 24.93 ± 0.2° in 2θ; and a DSC thermogram as substantially reported in Figure 12, wherein the main endothermic peaks fall at about 100°C-150°C and about 246°C-248°C. Said crystalline form is solvated with tetrahydrofuran.

HPLC purity: equal to or greater than 99.5%.

### Example 2: Preparation of bosentan sodium salt, crystalline form τ

By drying the ϕ form at 45-50°C under vacuum, the τ form is obtained. The crystalline solid presents a XRPD as substantially reported in Figure 6, wherein the most intense peaks fall at 6.27; 6.51; 8.34; 9.27; 9.78; 17.52; 18.09; 18.87; 19.68; 20.13; 21.69; 22.23; 22.98; 25.26; 26.28 ± 0.2° in 2θ; and a DSC thermogram as substantially reported in Figure 13, wherein the main endothermic peaks fall at about 80°C-130°C and about 246°C-248°C. Said crystalline form is solvated with tetrahydrofuran.

HPLC purity: equal to or greater than 99.5%.

### Example 3: Preparation of bosentan sodium salt, crystalline form ω

16 g of bosentan sodium salt form τ, 86 ml of acetone and 17 ml of methanol are loaded into a 250 ml jacketed reactor. The mixture is heated to the temperature of 35-40°C and maintained under said conditions for 35 minutes. 35 ml of methanol is added, and the mixture is heated to reflux temperature; the mixture is maintained in said conditions for 2 hours and then cooled to 20°C in 2 hours and maintained under said conditions for 12 hours. The mixture is cooled to 0°C in one hour and maintained under said conditions for 90 minutes. The product is filtered and washed twice with 6 ml of a 5:3 v/v mixture of acetone/methanol cooled to 0°C, and then with 11 ml of acetone cooled to 0°. Yield: 86.8%. The crystalline solid presents a XRPD as substantially reported in Figure 1, wherein the most intense peaks fall at 6.39, 8.31; 8.88; 9.30; 9.69; 16.71; 17.82; 18.03; 19.23; 20.22; 22.05; 23.07; 25.26; 25.77; 26.58 ± 0.2° in 2θ and a DSC thermogram as substantially reported in Figure 8, wherein the main endothermic peaks fall at about 70°C-110°C and 246°C-248°C. Said crystalline form is solvated with acetone and methanol.

HPLC purity: equal to or greater than 99.8%.

### Example 4: Preparation of bosentan sodium salt, crystalline form ω

40 g of bosentan sodium salt form D, 215 ml of acetone and 43 ml of methanol are loaded into a 500 ml jacketed reactor. The mixture is heated to the temperature of 35-40°C and maintained under said conditions for 35 minutes. 86 ml of methanol are added, and the mixture is heated to reflux temperature; the mixture is maintained in said conditions for 2 hours and then cooled to the temperature of 25°C in 2 hours and seeded with 400 mg of bosentan sodium salt form ψ. The mixture is maintained under stirring at 25°C for 21 hours. The mixture is cooled to 0°C in one hour and maintained under said conditions for 90 minutes. The product is filtered and washed twice with 15 ml of a 5:3 v/v mixture of acetone/methanol cooled to 0°C, and then with 25 ml of acetone cooled to 0°. Yield: 89%. The crystalline solid presents a XRPD as substantially reported in Figure 1, wherein the most intense peaks fall at 6.39, 8.31; 8.88; 9.30; 9.69; 16.71; 17.82; 18.03; 19.23; 20.22; 22.05; 23.07; 25.26; 25.77; 26.58 ± 0.2° in 2θ and a DSC thermogram as substantially reported in Figure 8, wherein the main endothermic peaks fall at about 70°C-110°C and 246°C-248°C. Said crystalline form is solvated with acetone and methanol.

HPLC purity: equal to or greater than 99.8%.

### Example 5: Preparation of bosentan sodium salt, crystalline form ψ

By drying the ω form at 45-50°C under vacuum, the ψ form is obtained.

The crystalline solid presents a XRPD as substantially reported in Figure 2, wherein the most intense peaks fall at 6.39; 8.28; 9.72; 11.4; 13.86; 16.77; 17.43; 18.12; 18.90; 10.22; 22.20; 22.86; 24.63; 26.16; 27.78 ± 0.2° in 2θ; and a DSC thermogram as substantially reported in Figure 9, wherein the melting endotherm falls at about 246°C-248°C; with a water content of between about 0.1 and 1.0%, so that it can be described as substantially anhydrous.

HPLC purity: greater than 99.8%.

### Example 6: Preparation of bosentan sodium salt, crystalline form χ

20 g of bosentan sodium salt form D, 108 ml of acetone and 21 ml of methanol are loaded into a 250 ml jacketed reactor. The mixture is heated at the temperature of 40°C and maintained under said conditions for 35 minutes. 43 ml of methanol are added, and the mixture is heated to reflux temperature; the mixture is maintained in said conditions for 2 hours and then cooled to 25°C in 3 hours and maintained under said conditions for 14 hours. The product is filtered and washed twice with 8 ml of a 5:3 v/v mixture of acetone/methanol cooled to 0°C, and then with 13 ml of acetone cooled to 0°. Yield: 75%. The crystalline solid presents a XRPD as substantially reported in Figure 3, wherein the most intense peaks fall at 5.34; 6.03; 9.12; 11.16; 12.27; 15.18; 15.57; 16.02; 16.20; 16.47; 16.89; 18.54; 20.64; 22.65; 26.85 ± 0.2° in 2θ and a DSC thermogram as substantially reported in Figure 10, wherein the melting endotherm falls at about 210°C-230°C;

HPLC purity: exceeding 98%.

### Example 7: Preparation of bosentan sodium salt crystalline form σ

By drying the χ form at 45-50°C under vacuum, the σ form is obtained.

The crystalline solid presents a XRPD as substantially reported in Figure 4, wherein the most intense peaks fall at 5.31, 6.18; 6.96; 9.27; 10.68; 12.21; 12.57; 13.83; 15.48; 18.09; 18.69; 19.23; 20.61; 21.69; 24.39 ± 0.2° in 2θ and a DSC thermogram as substantially reported in Figure 11, wherein the melting endotherm falls at about 210°C-230°C; and a water content of between about 0.1 and 1.0%, so that it can be described as substantially anhydrous.

HPLC purity: exceeding 98%.

### Example 8: Preparation of bosentan in the monohydrate crystalline form

The product bosentan sodium salt in crystalline form ω, obtained according to example 3 or 4 above, is suspended in 500 ml of ethanol and then slowly acidified with 35 g of 37% hydrochloric acid to a pH of 2-3, the temperature being maintained at under 30°C. The mixture is slowly diluted with water (475 ml) and left under stirring at ambient temperature for about 3 hours. The solid is filtered and washed with a 1:1 ethanol/water mixture (2 x 75 ml), and the product is filtered at 25-30°C at low pressure. 183 g of product is obtained, with a yield of 95%. The resulting product has an HPLC purity of about 99.9%, and a mean particle diameter D₅₀ of about 15 microns. Said product is in crystalline form, having a XRPD as reported in Figure 1 of EP 2 072 503 and a DSC tracing as reported in Figure 2 of EP 2 072 503. It also has a water content of between about 2.5 and about 3.5%.

### Example 9: Preparation of bosentan of formula (I), in the monohydrate crystalline form

45.7 g of bosentan sodium salt crystalline form ϕ, as obtainable according to example 1, 46 ml of water and 144 g of ethanol are loaded into a 500 ml jacketed reactor. The reaction mixture is heated to 40°C, and 3.6 g of 37% HC1 is added. The mixture is then heated to the reflux temperature and maintained under said conditions for 1 hour. The solution obtained is filtered hot, and the reactor and filter are washed with 36 g of ethanol heated to 40-45°C. The solution is distilled under vacuum until 105 ml of distillate is obtained. The mixture is cooled to 40°C, and 3.6 g of 37% HCl is added. The mixture is maintained under stirring for 30 minutes and then cooled to 25°C, and maintained at said temperature for 30 minutes. 55 ml of water is added, and the mixture is maintained under stirring for 2 hours. The mixture is filtered and the crystalline product thus obtained is washed with 70 ml of a 2:1 water/ethanol solution. Yield: 94%.

The resulting product has a purity of about 99.9%, and a mean particle diameter D₅₀ of about 15 microns. Said product is in crystalline form, having a XRPD as reported in Figure 1 of EP 2 072 503, a DSC thermogram as reported in Figure 2 of EP 2 072 503, and a water content between about 2.5 and about 3.5%.

Proceeding in a similar way to that described in the above example 8 or 9, starting with a crystalline form selected from the group comprising Forms ψ, χ, σ, ϕ, τ and ω, which is not the same crystalline form of bosentan sodium salt as used in said examples, bosentan is obtained in the monohydrate crystalline form, having the same XRPD and DSC characteristics as the product obtained according to example 8 or 9.

## Claims

1. A process for the purification of bosentan of formula (I), in particular in monohydrate form, or a salt thereof 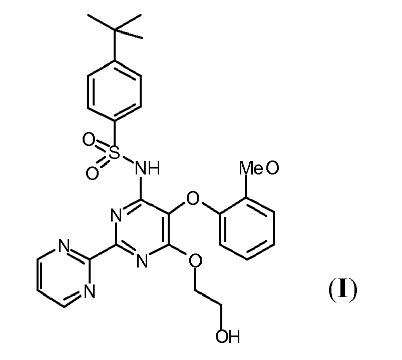 comprising:
a) preparing a crystalline form of bosentan sodium salt of formula (I') 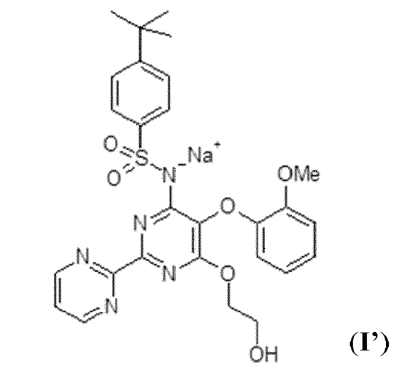 selected from the group comprising:
i. a crystalline form solvated with acetone and methanol, herein defined as Form ω, having a XRPD as substantially reported in Figure 1, wherein the most intense peaks fall at 6.39, 8.31; 8.88; 9.30; 9.69; 16.71; 17.82; 18.03; 19.23; 20.22; 22.05; 23.07; 25.26; 25.77; 26.58 ± 0.2° in 2θ; and a DSC thermogram as substantially reported in Figure 8 wherein the main endothermic peaks fall at about 70°C-110°C and at about 246°C-248°C;
ii. a crystalline form herein defined as Form ψ, having a XRPD as substantially reported in Figure 2, wherein the most intense peaks fall at 6.39; 8.28; 9.72; 11.4; 13.86; 16.77; 17.43; 18.12; 18.90; 10.22; 22.20; 22.86; 24.63; 26.16; 27.78 ± 0.2° in 2θ; and a DSC thermogram as substantially reported in Figure 9, wherein the melting endotherm falls at about 246°C-248°C;
iii. a crystalline form, herein defined as Form χ, having a XRPD as substantially reported in Figure 3, wherein the most intense peaks fall at 5.34; 6.03; 9.12; 11.16; 12.27; 15.18; 15.57; 16.02; 16.20; 16.47; 16.89; 18.54; 20.64; 22.65; 26.85 ± 0.2° in 2θ, and having a DSC thermogram as substantially reported in Figure 10, wherein the melting endotherm falls at about 210°C-230°C;
iv. a crystalline form, herein defined as Form σ, having a XRPD as substantially reported in Figure 4, wherein the most intense peaks fall at 5.31, 6.18; 6.96; 9.27; 10.68; 12.21; 12.57; 13.83; 15.48; 18.09; 18.69; 19.23; 20.61; 21.69; 24.39 ± 0.2° in 2θ, and a DSC thermogram as substantially reported in Figure 11, wherein the melting endotherm falls at about 210°C-230°C;
v. a crystalline form solvated with tetrahydrofuran, herein defined as Form ϕ, having a XRPD as substantially reported in Figure 5, wherein the most intense peaks fall at 4.95; 6.09; 8.16; 9.12; 9.99; 16.44; 17.94; 18.69; 20.28; 21.51; 22.83 and 24.93 ± 0.2° in 2θ; and a DSC thermogram as substantially reported in Figure 12, wherein the main endothermic peaks fall at about 100°C-150°C and at about 246°C-248°C; and
vi. a crystalline form solvated with tetrahydrofuran, herein defined as Form τ, having a XRPD as substantially reported in Figure 6, wherein the most intense peaks fall at 6.27; 6.51; 8.34; 9.27; 9.78; 17.52; 18.09; 18.87; 19.68; 20.13; 21.69; 22.23; 22.98; 25.26; 26.28 ± 0.2° in 2θ; and having a DSC thermogram as substantially reported in Figure 13, wherein the main endothermic peaks fall at about 80°C -130°C and at about 246°C-248°C;
b) forming a dispersion of a crystalline form of bosentan sodium salt as defined above in ethanol, optionally a mixture with water;
c) treating the dispersion thus obtained with an organic or inorganic acid or a solution thereof; and
d) recovering bosentan of formula **(I),** in particular in monohydrate form, and optionally converting it to a salt thereof.

2. A process according to claim 1, wherein a crystalline form is prepared by a process comprising:
- forming a solution of bosentan sodium salt in tetrahydrofuran or a mixture thereof with at least one additional solvent;
- cooling the solution thus obtained to obtain a precipitate; and
- recovering the precipitate thus obtained.

3. A process according to claim 1, wherein bosentan sodium salt in crystalline form τ is prepared by a process comprising drying bosentan sodium salt in crystalline form ϕ, as defined in claim 1.

4. A process according to claim 1, wherein bosentan sodium salt in crystalline form ω is prepared by a process comprising:
- dispersing bosentan sodium salt in crystalline Form τ, as defined in claim 1, in a solvent mixture comprising at least acetone and methanol to obtain a dispersion of bosentan sodium salt;
- cooling the dispersion; and
- recovering the solid thus obtained; or, by a process comprising seeding a dispersion containing bosentan sodium salt, for example in crystalline form D, with a seed of crystalline form ω as previously obtained, or by seeding with a seed of crystalline form ψ, as defined in claim 1.

5. A process according to claim 1, wherein bosentan sodium in crystalline Form ψ is prepared by a process comprising drying bosentan sodium salt in crystalline form ω, as defined in claim 1.

6. A process according to claim 1, wherein bosentan sodium salt in crystalline Form χ is prepared by a process comprising:
- dispersing bosentan sodium salt in crystalline form D in a solvent mixture comprising a C₃-C₇ ketone and a C₁-C₇ alkanol;
- cooling the dispersion; and
- recovering the solid thus obtained.

7. A process according to claim 1, wherein bosentan sodium salt in crystalline form σ is prepared by a process comprising drying bosentan sodium salt in crystalline form χ, as defined in claim 1.

8. A process according to claim 1, wherein a dispersion of bosentan sodium salt in a crystalline form, as defined in claim 1, is formed at a temperature ranging between about 0°C and the reflux temperature of the solvent.

9. A process according to claim 1, wherein an organic or inorganic acid is selected from the group comprising a C₁-C₈ carboxylic acid, a sulphonic acid, sulphuric acid or a hydrohalic acid, preferably hydrochloric acid.

10. Bosentan sodium salt in crystalline form selected from the group comprising:
i. a crystalline form solvated with acetone and methanol, herein defined as Form ω, having a XRPD as substantially reported in Figure 1, wherein the most intense peaks are at 6.39, 8.31; 8.88; 9.30; 9.69; 16.71; 17.82; 18.03; 19.23; 20.22; 22.05; 23.07; 25.26; 25.77; 26.58 ± 0.2° in 2θ and a DSC thermogram as substantially reported in Figure 8, wherein the main endothermic peaks fall at about 70°C-110°C and at about 246°C-248°C;
ii. a crystalline form herein defined as Form ψ, having a XRPD as substantially reported in Figure 2, wherein the most intense peaks fall at 6.39; 8.28; 9.72; 11.4; 13.86; 16.77; 17.43; 18.12; 18.90; 10.22; 22.20; 22.86; 24.63; 26.16; 27.78 ± 0.2° in 2θ; and a DSC thermogram as substantially reported in Figure 9, wherein the melting endotherm falls at about 246°C-248°C;
iii. a crystalline form, herein defined as Form χ, having a XRPD as substantially reported in Figure 3, wherein the most intense peaks fall at 5.34; 6.03; 9.12; 11.16; 12.27; 15.18; 15.57; 16.02; 16.20; 16.47; 16.89; 18.54; 20.64; 22.65; 26.85 ± 0.2° in 2θ and having a DSC thermogram as substantially reported in Figure 10, wherein the melting endotherm falls at about 210°C-230°C;
iv. a crystalline form, herein defined as Form σ, having a XRPD as substantially reported in Figure 4, wherein the most intense peaks fall at 5.31, 6.18; 6.96; 9.27; 10.68; 12.21; 12.57; 13.83; 15.48; 18.09; 18.69; 19.23; 20.61; 21.69; 24.39 ± 0.2° in 2θ and a DSC thermogram as substantially reported in Figure 11, wherein the melting endotherm falls at about 210°C-230°C;
v. a crystalline form solvated with tetrahydrofuran, herein defined as form ϕ, having a XRPD as substantially reported in Figure 5 wherein the most intense peaks fall at 4.95; 6.09; 8.16; 9.12; 9.99; 16.44; 17.94; 18.69; 20.28; 21.51; 22.83 and 24.93 ± 0.2° in 2θ; and a DSC thermogram as substantially reported in Figure 12 wherein the main endothermic peaks fall at about 100°C-150°C and at about 246°C-248°C; and
vi. a crystalline form solvated with tetrahydrofuran, herein defined as Form τ, having a XRPD as substantially reported in Figure 6, wherein the most intense peaks fall at 6.27; 6.51; 8.34; 9.27; 9.78; 17.52; 18.09; 18.87; 19.68; 20.13; 21.69; 22.23; 22.98; 25.26; 26.28 ± 0.2° in 2θ; and having a DSC thermogram as substantially reported in Figure 13 wherein the main endothermic peaks fall at about 80°C-130°C and at about 246°C-248°C.
